Europäisches Patentamt

European Patent Office

Office européen des brevets

(19)

(11) Veröffentlichungsnummer: **0 223 110 B1**

(12) **EUROPÄISCHE PATENTSCHRIFT**

(45) Veröffentlichungstag der Patentschrift: **02.01.92**

(51) Int. Cl.⁵: **C12N 13/00**, C12P 5/02

(21) Anmeldenummer: **86114885.6**

(22) Anmeldetag: **27.10.86**

(54) **Verfahren zur stoffwechsel- und/oder wachstumssteigernden Behandlung von Mikroorganismen sowie Vorrichtung zur Durchführung des Verfahrens.**

(30) Priorität: **26.10.85 DE 3538194**

(43) Veröffentlichungstag der Anmeldung:
**27.05.87 Patentblatt 87/22**

(45) Bekanntmachung des Hinweises auf die
Patenterteilung:
**02.01.92 Patentblatt 92/01**

(84) Benannte Vertragsstaaten:
**AT BE CH DE ES FR GB IT LI LU NL SE**

(56) Entgegenhaltungen:
CH-A- 495 772            DE-A- 2 534 757
DE-A- 2 841 455          FR-A- 1 410 428
FR-A- 2 059 215          FR-A- 2 336 480
FR-A- 2 554 827          US-A- 3 095 359
US-A- 4 487 834

**SOVIET INVENTIONS ILLUSTRATED, Section
Chemical, Woche K34, 5. Oktober 1983, Zusammenfassung Nr. 83-746169/34, Derwent
Publications Ltd; & SU-A-968 071
(VETERINARY SANITARY) 20-02-1981**

Idem

(73) Patentinhaber: **Doevenspeck, Heinz
Sigurdstrasse 1
W-4950 Minden(DE)**

(72) Erfinder: **Doevenspeck, Heinz
Sigurdstrasse 1
W-4950 Minden(DE)**

(74) Vertreter: **Bolte, Erich, Dipl.-Ing. et al
c/o Meissner, Bolte & Partner Patentanwälte
Hollerallee 73
W-2800 Bremen 1(DE)**

## Beschreibung

Die Erfindung betrifft ein Verfahren zur stoffwechsel- und/oder wachstumssteigernden Behandlung von Mikroorganismen, wobei die Mikroorganismen elektrischen Feldern mit einer impulsförmig verlaufenden Feldstärke (E-Impulse) von bis zu 3,5 kV/cm ausgesetzt werden. Weiterhin betrifft die Erfindung eine Vorrichtung zur Durchführung des Verfahrens.

Es ist bekannt, daß elektrische Felder auf Mikroorganismen - Bakterien, Pilze - einen Einfluß besitzen. Wenn man nämlich Bakterien elektrischen Feldern aussetzt, deren zeitlicher Verlauf als impulsförmig beschrieben werden kann, so wurde nachgewiesen, daß Feldstärken von 6 bis 20 kV/cm einen außerordentlich starken letalen Effekt auf die Bakterien ausüben (s. z.B. Hülsheger et al: Radiation and Environmental Biophysics [1981] 20:53 - 65; und a.a.O. [1980] 18:281 - 288).

In vielen Fällen will man aber die Mikroorganismen nicht töten, sondern gerade im Gegenteil kultivieren, wenn man beispielsweise Abwässer mittels mikrobieller Verfahren klären will, so geht es gerade darum, diese Mikroorganismen, welche die abzubauenden Substanzen metabolisieren, zu kultivieren. Um dies zu erreichen, ist es bekannt, den Abwässern bestimmte Stoffe zuzusetzen, um z.B. nitrifikanten Wasserstoff, der für deren Metabolismus benötigt wird, zur Verfügung zu stellen. Darüber hinaus betreibt man die Verfahren nach Möglichkeit bei Temperaturen, bei denen die Mikroorganismen möglichst hohe Stoffwechsel- und Vermehrungsraten haben. In vielen Fällen reicht dies jedoch nicht aus, um ökonomisch wirkungsvoll den gewünschten Prozeß in Gang zu halten.

Aus der französischen Offenlegungsschrift FR-2 554 827 ist ein Verfahren zur Steigerung der Stoffwechsel- und Vermehrungsraten von Mikroorganismen bekannt, bei dem man die Mikroorganismen einem impulsförmig verlaufenden elektrischen Feld aussetzt. Hierbei wird ein möglicher Einfluß der Impulsform auf die erreichbare Steigerung der Stoffwechsel- und Vermehrungsraten der Mikroorganismen nicht erkannt.

Ausgehend vom obengenannten Stand der Technik ist es Aufgabe der vorliegenden Erfindung, die Effektivität des bekannten Verfahrens zur Stoffwechsel- und/oder wachstumssteigernden Behandlung von Mikroorganismen zu erhöhen.

Diese Aufgabe wird erfindungsgemäß dadurch gelöst, daß die Feldstärke der elektrischen Felder innerhalb von höchstens 1 ms auf den Maximalwert ansteigt und exponentiell abfällt. Es wurde gefunden, daß durch die erfindungsgemäße Gestaltung der Impulsform eine besonders starke Wachstumssteigerung bei den Mikroorganismen erzielbar ist. Diese Wachstumssteigerung kann man insbesondere an deren Stoffwechselraten nachweisen. Die erfindungsgemäße Impulsform entsteht dann, wenn man einen aufgeladenen Kondensator über einen Schalter an einen Verbraucherwiderstand anschließt, über den er sich dann entlädt. Dieses hat sich als besonders wirkungsvoll erwiesen.

Vorteilhafterweise läßt man die Feldstärke innerhalb von 100 $\mu$s, vorzugsweise innerhalb von 50 $\mu$s, auf den Maximalwert ansteigen. Es kommt also darauf an, daß der Anstieg (innerhalb gewisser Grenzen) möglichst steil verläuft. Den Abfall (entlang einer e-Funktion) läßt man vorteilhafterweise innerhalb von 10 - 100 $\mu$s vonstatten gehen.

Besonders wirkungsvoll ist das Verfahren dann, wenn man die Feldstärke in ihrem Maximalwert bis auf 0,1 - 1 kV/cm, insbesondere aber auf 0,2 - 0,5 kV/cm begrenzt.

Weiterhin ist es besonders vorteilhaft, wenn die Feldstärkenrichtung in bezug auf die Strömungsrichtung der zu behandelnden Flüssigkeit wechselt. Dies bietet sich vor allem bei größeren Anlagen mit mehreren, hintereinandergeschalteten Elektroden-Gruppen an, wo zur Feldstärkenrichtungsänderung mehrere Elektrodensätze hintereinander angeordnet, also in Reihe geschaltet sind. Alternativ kann die Änderung der Feldstärkenrichtung auch durch eine Umpolung des (Impuls-)Generators erfolgen, so daß die aufeinanderfolgenden Impulse unterschiedliche Feldstärkenrichtungen aufweisen. Dieses bietet sich vor allem für kleinere Anlagen an.

Alternativ kann die Feldstärkenrichtung konstant gehalten, also mit Gleichstrom-Pulsen gearbeitet werden. Dadurch, daß die Entladungen lediglich pulsförmig sind, ist hierbei der Elektrolyseeffekt im wesentlichen zu vernachlässigen. Auch thermische Effekte sind aufgrund der geringen, der Flüssigkeit zugeführten Energie im wesentlichen zu vernachlässigen.

Bei einer bevorzugten Ausführungsform des Verfahrens wendet man die E-Impulse mit Repititionsraten an, die niedrig sind - gemessen an den Impulsdauern. Das heißt, man macht zwischen den einzelnen E-Impulsen Pausen. Besonders vorteilhaft ist es hierbei, wenn die Repititionsraten zwischen 5 und 15 Hz betragen.

Vorteilhafterweise wendet man die E-Impulse nur in Pulsgruppen, also nicht als kontinuierlichen Pulszug an und macht zwischen derartigen Pulsgruppen Pausen. Überraschenderweise hat es sich nämlich gezeigt, daß derartige Pausen nicht etwa zu einer Verminderung der Wirkung der E-Impulse, sondern vielmehr zu einer Steigerung der Wirkung führen. Vorzugsweise wählt man die Pulsgruppen- bzw. Pausenintervalle zwischen den Pulsgruppen kürzer als jeweils 30 min, vorzugsweise kürzer als 15 min.

Eine besonders bevorzugte Verwendung für das Verfahren ist die mikrobielle Erzeugung von Methangas bzw. Biogas (Faulgas) bzw. die Behandlung von Abwässern in anaerobe gefahrenen Reaktoren. Neben der erwähnten wachstumssteigernden bzw. stoffwechselfördernden Wirkung der E-Impulse hat es sich überraschenderweise gezeigt, daß dann, wenn man nach dem erfindungsgemäßen Verfahren vorgeht, die Erzeugung von flüchtigen Schwefelverbindungen drastisch reduziert wird. Im allgemeinen ist es nämlich so, daß das normale Biogas einen beträchtlichen Anteil an Schwefelwasserstoff oder anderen flüchtigen Schwefelverbindungen enthält, die man erst aus dem Gas entfernen muß, bevor man dies z.B. in Gasmotoren weiterverwenden kann. Bei der Anwendung des erfindungsgemäßen Verfahrens auf die Biogaserzeugung sind derartige Schwefelverbindungen nun - falls überhaupt -, so doch nur in drastisch reduzierten Konzentrationen im erzeugten Gas nachweisbar, so daß dieses nicht nur in erhöhter Menge (aufgrund der Anwendung des erfindungsgemäßen Verfahrens), sondern auch in einer wesentlich leichter verwendbaren Qualität, also in besserer Qualität erzeugt wird, als dies bisher der Fall war. Selbst dann, wenn man das erfindungsgemäße Verfahren nicht bei seinen optimalen Parametern fährt (Pulsamplitude, Pulsdauer, Pulsfrequenz etc.), so wird dennoch durch die Anwendung des Verfahrens die Biogaserzeugung hinsichtlich ihres Wirkungsgrades wesentlich verbessert, da eben die genannten Schadstoffe nur in reduzierten Mengen im Gas auftreten.

Eine weitere wesentliche Verwendungsmöglichkeit des erfindungsgemäßen Verfahrens ist die mikrobiologische Erzeugung von Enzymen oder anderer nicht gasförmiger Produkte.

Zur Durchführung des Verfahrens eignet sich eine Vorrichtung mit mindestens einem Reaktor mit Zulauf- und Ablaufleitung. Diese Vorrichtung weist hierbei mindestens ein Paar von Kondensator-Elektroden auf, die derart angeordnet sind, daß sich mindestens Teilmengen des Reaktorinhalts mindestens zeitweise zwischen den Elektroden befinden. Diese Elektrodenplatten sind mit einer impulserzeugenden Generatorschaltung zum Versorgen mit einer elektrischen Spannung verbunden. Die Generatorschaltung umfaßt dabei ein Netzteil und mindestens einen Speicherkondensator. Weiterhin ist ein Umschalter vorgesehen, der mit dem Netzteil und dem Speicherkondensator sowie mit den Elektroden derart verschaltet ist, daß in einer ersten Schalterstellung der Speicherkondensator vom Netzteil geladen und in einer zweiten Schalterstellung über die Elektroden entladen wird. Diese relativ einfache Schaltung ist dennoch sehr wirkungsvoll, da den Mikroorganismen die Pulse in der optimalen Form zugeführt werden. Man kann den Umschalter zwar mechanisch steuern, jedoch ist es von besonderem Vorteil, wenn man diesen Umschalter elektrisch steuern läßt. Derartige elektrische Umschalter wären beispielsweise Ignitrons, Thyristoren, Transistoren usw.

Diese elektrisch steuerbaren Schalter verbindet man vorteilhafterweise mit einem Steueroszillator. Besonders einfach ist die Vorrichtung dann, wenn der Umschalter aus zwei An/Aus-Schaltelementen besteht, wobei das erste Schaltelement zwischen dem Speicherkondensator und dem Netzteil, das zweite Schaltelement zwischen Kondensator und Elektroden angeordnet ist. Man betreibt hierbei die Vorrichtung so, daß man zunächst den zweiten Schalter (Verbindung zwischen Speicherkondensator und Elektroden) abschaltet, das erste Schaltelement aber anschaltet, so daß der Kondensator vom Netzgerät aufgeladen wird. Sobald der Kondensator geladen ist, öffnet man den ersten Schalter, so daß der Kondensator nunmehr "mit einem Bein in der Luft hängt". Sodann schließt man den zweiten Schalter und koppelt somit den Kondensator an die Elektroden. Auf diese Weise bekommt man exakt definierte Ein- und Ausschaltverläufe.

Vorteilhafterweise weist der Steueroszillator einen Pulsgenerator auf, dessen Ausgangspulse über Steuermittel von einem Generator periodisch gesperrt werden können. Die Ausgangspulse des Pulsgenerators bewirken den Umschaltvorgang zwischen Aufladen und Entladen des Speicherkondensators, wobei während der Zeit, in der die Ausgangspulse des Pulsgenerators von dem (weiteren) Generator gesperrt sind, die Kondensatorplatten nicht mehr an den Kondensator gekoppelt werden.

Man kann bei der erfindungsgemäßen Vorrichtung die Elektroden im Reaktorgehäuse anordnen, so daß sich der gesamte Reaktorinhalt ständig zwischen den Platten befindet. Vorzugsweise sind allerdings die Elektroden in einem strommäßig separaten Gehäuse so angeordnet, daß der Zwischenraum zwischen den Elektroden über Leitungsmittel in Strömungsverbindung mit dem Innenraum des Reaktors steht. Der Reaktorinhalt wird also durch den Elektrodenzwischenraum geleitet, so daß sich nur ein Teilvolumen des Reaktorinhalts ständig zwischen den Platten befindet. Der Hauptanteil des Reaktorvolumens ist somit im wesentlichen feldfrei, wobei selbstverständlich sowohl über die flüssigkeitsmäßige Kopplung zwischen den Elektroden und dem Reaktorinnenraum als auch über die elektromagnetische Kopplung Einstreuungen der zwischen den Elektroplatten erzeugten Felder in den Reaktorinnenraum möglich (bzw. wahrscheinlich) sind.

Man kann hierbei den Umlauf dadurch erzeugen, daß man die Leitungen (Zu/Ableitung) zwischen Reaktor und Elektrodengehäuse entsprechend anordnet, vorzugsweise wird allerdings eine Umwälzpumpe in eine der Leitungen eingesetzt.

Bei einem bevorzugten Ausführungsbeispiel der erfindungsgemäßen Vorrichtung sind mehrere Elektroden strömungsmäßig hintereinander angeordnet, so daß die durchgeleitete Flüssigkeit (mit Mikroorganismen) einen längeren Raum zwischen den Elektroden durchquert, sich also eine längere Zeit zwischen den Elektroden befindet. Man kann hierbei die Elektroden parallelschalten, um zwischen allen Elektrodenpaaren die gleichen Spannungsverläufe zu erzeugen, aber auch hintereinanderschalten, wenn unterschiedliche Spannungsverläufe zwischen den Elektrodenpaaren erzeugt werden sollen.

Vorteilhafterweise sind die Elektroden im Gehäuse derart angeordnet, daß sie ein Labyrinth bilden, durch welches die Flüssigkeit strömt. Auf diese Weise gelangt man zu einem besonders kompakten Gehäuse, bei dem dennoch eine längere Behandlung der durchgeleiteten Flüssigkeit gewährleistet ist.

Bei einer weiteren bevorzugten Ausführungsform der Erfindung sind die Elektroden koaxial zueinander angeordnet. Diese Ausführungsform der Erfindung führt zu einem kompakten, besonders strömungsgünstigen Gehäuse, das nur wenige Ruhezonen (strömungsmäßig gesehen) aufweist, in denen sich Feststoffe anlagern könnten.

Bei einer anderen bevorzugten Ausführungsform der Erfindung weisen die Elektroden Durchlässe auf und sind derart im Gehäuse zueinander angeordnet, daß sie im wesentlichen senkrecht zu ihrer Oberfläche durchströmt werden. Bei einer entsprechenden Anordnung der Öffnungen im Gehäuse kann man auch hier einen Strömungsverlauf schaffen, der im wesentlichen frei von Ruhezonen bzw. Ablagerungsflächen ist.

Vorteilhafterweise führt man den Zulauf und den Ablauf des Reaktors über eine gemeinsame Elektrodenstrecke. Dies ist insbesondere dann der Fall, wenn die Temperatur des Reaktorinhaltes von der Temperatur der zugeleiteten Flüssigkeit abweicht. In diesem Fall fördert dies nämlich den Metabolismus der Mikroben, wenn man das frisch zugeleitete Substrat im wesentlichen auf die Reaktortemperatur vorwärmt, damit die Mikroorganismen keinen Temperaturschock erleiden. Dann, wenn man beispielsweise thermophile Mikroben im Reaktor halten will, also Mikroben, die in Temperaturbereichen bis zu 70, 80 °C (mit hohen Stoffwechselraten) arbeiten, ist es von besonders großem Vorteil, wenn man eine Elektrodenstrecke zwischen Zulauf- und Ablaufleitung einführt.

Weiterhin ist es von großem Vorteil, wenn man in die Zulauf- und in die Ablaufleitung Entkeimungsvorrichtungen einbaut. Eine Entkeimungsvorrichtung im Zulauf bewirkt, daß man den Reaktorinhalt nur mit den Mikroorganismen "impfen" kann, die man tatsächlich im Reaktor haben will, während durch die Abwässer zugeführte Fremd-Mikroorganismen abgetötet werden. Was die Mikroorganismen betrifft, welche aus der Ablaufleitung in die weiteren, nachgeschalteten Anlagen gelangen, so ist es ebenfalls von Vorteil, diese von den nachgeschalteten Anlagen fernzuhalten.

Bei einer besonders bevorzugten Ausführungsform der erfindungsgemäßen Vorrichtung ist die Entkeimungsvorrichtung in die Elektrodenstrecke integriert. Die Elektrodenstrecke besteht hierbei - ähnlich wie die Vorrichtung zur Steigerung des Wachstums - aus Kondensatorplatten, die an eine impulsabgebende Vorrichtung angeschlossen sind. In diesem Fall waren jedoch die Impulse so stark gewählt (hinsichtlich ihrer maximalen Feldstärke), daß die eingangs erwähnte letale Wirkung auf die Mikroorganismen eintritt.

Weitere bevorzugte Ausführungsformen der Erfindung ergeben sich aus den nachfolgend beschriebenen Ausführungsbeispielen, die anhand von Abbildungen näher erläutert werden. Hierbei zeigt:

Fig. 1 eine prinzipielle Anordnung zur Durchführung des erfindungsgemäßen Verfahrens,

Fig. 2 eine erste bevorzugte Ausführungsform der Elektroden-Vorrichtung im Längsschnitt,

Fig. 3 die Elektroden-Vorrichtung gemäß der Fig. 2 mit zwei Elektrodensätzen,

Fig. 4 eine zweite bevorzugte Ausführungsform der Elektroden-Vorrichtung im Längsschnitt,

Fig. 5 eine dritte bevorzugte Ausführungsform der Elektroden-Vorrichtung im Längsschnitt,

Fig. 6 die Elektroden-Vorrichtung gemäß der Fig. 5 mit doppelpoligen Elektroden,

Fig. 7 eine bevorzugte Ausführungsform einer Elektrodenstrecke im Längsschnitt,

Fig. 8 die Elektrodenstrecke gemäß der Fig. 7 mit zwei hintereinanderliegenden Elektrodensätzen,

Fig. 9 eine erste (Prinzip-)Schaltung zur Durchführung des erfindungsgemäßen Verfahrens, und

Fig. 10 eine zweite schematisierte Darstellung, welche im wesentlichen die in Fig. 6 gezeigten Funktionen aufweist.

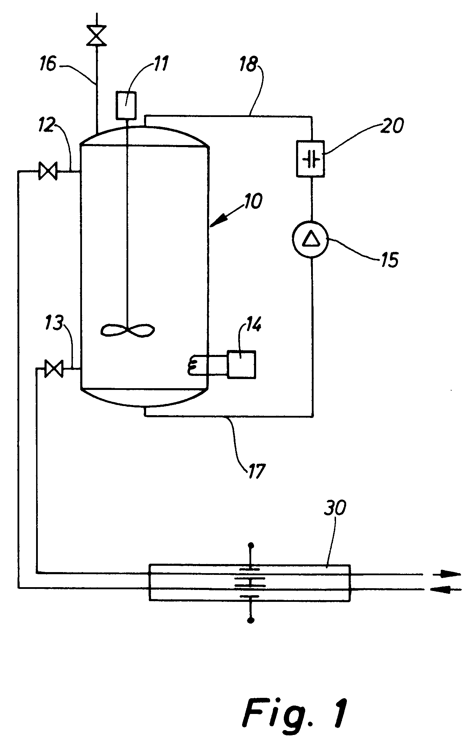

Wie aus Fig. 1 hervorgeht, weist der Reaktor 10 zur Durchführung des erfindungsgemäßen Verfahrens ein Reaktorgehäuse auf, in dem sich (in üblicher Weise) ein Rührwerk 11 befindet. Weiterhin ist mit dem Reaktorinnenraum eine Heizung 14 gekoppelt, die beispielsweise aus einem Gasbrenner gespeist werden kann, der mit Biogas versorgt wird, was aus einer Gas-Ausgangsleitung 16 des Reaktors 10 dem Reaktorinneren entnommen wird.

Der Reaktor 10 ist mit einer Zulaufleitung 12 (mit Ventil) sowie mit einer Ablaufleitung 13 versehen. Zulauf- und Ablaufleitung 12/13 sind durch einen Wärmetauscher 30 im Gegenstrom geführt.

Weiterhin ist der Reaktor 10 an seinem Boden mit einer Umlaufableitung 17 versehen, welche in den Saugeingang einer Umwälzpumpe 15 geführt ist. Der Druckausgang der Umwälzpumpe 15 führt in den Eingang einer Elektroden-Vorichtung 20, die im folgenden näher beschrieben werden wird. Der Ausgang der Elektroden-Vorrichtung 20 steht über eine Umlaufrückleitung 18 mit dem Deckel des Reaktors 10 in Verbindung. Auf diese Weise ist bei Betrieb des Rührwerks 11 und der Umwälzpumpe 15 gewährleistet, daß der Reaktorinhalt gleichmäßig (über die Zeit gesehen) durch die Elektroden-Vorrichtung 20 hindurchgeleitet wird.

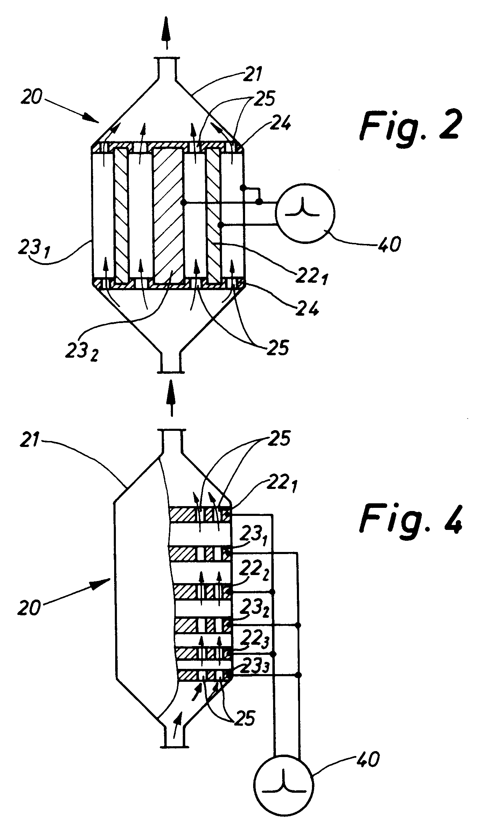

In Fig. 2 ist eine bevorzugte Ausführungsform der Elektroden-Vorrichtung 20 gezeigt. Hierbei ist das Gehäuse 21 der Elektroden-Vorrichtung 20 aus leitendem Werkstoff hergestellt. Im Gehäuse 21 befinden sich in horizontaler Anordnung zwei isolierende Elektrodenhalter 24 mit Durchlässen 25. In den Elektrodenhaltern 24 sind eine zentrale Elektrode $23_2$ und eine mittlere Elektrode $22_1$ gehalten. Die Durchlässe 25 sind hierbei so angeordnet, daß sie die Räume zwischen der Außenwand des Gehäuses 21 und mittlerer Elektrode $22_1$ bzw. mittlerer Elektrode $22_1$ und zentraler Elektrode $23_2$ mit der Umlaufableitung 17 und der Umlaufrückleitung 18 der Vorrichtung 20 verbinden.Strömungsmäßig gesehen stehen somit die beiden Koaxial-Räume in Parallelschaltung, während in elektrischer Hinsicht gesehen zwei parallelgeschaltete Elektroden vorliegen, da die zentrale Elektrode $23_2$ mit dem Gehäuse 21, das die äußerste Elektrode $23_1$ bildet, elektrisch verbunden ist, während die mittlere Elektrode $22_1$ die Gegenelektrode für beide so gebildete Elektroden bildet. Dieses Paar von Elektroden ist mit einem Generator 40 verbunden, der weiter unten näher beschrieben wird.

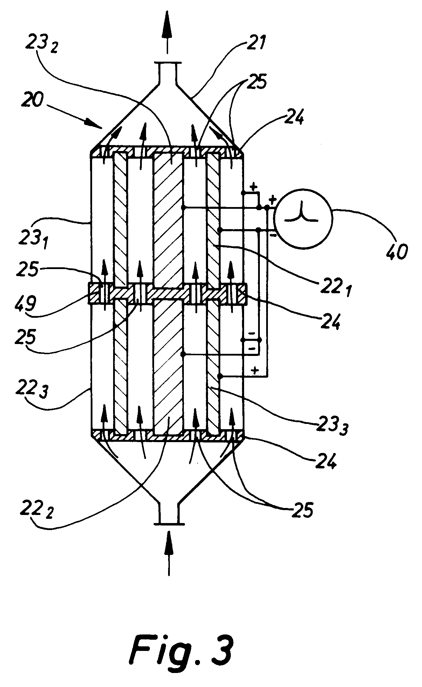

Die Fig. 3 zeigt die Elektrodenvorrichtung 20 mit zwei hintereinanderliegend angeordneten Elektrodensätzen. Ein (oberer) Elektrodensatz wird demnach gebildet durch die positiven Elektroden $23_1$ und $23_2$ sowie eine negative Elektrode $22_1$. Der (untere) Elektrodensatz besteht aus einer positiven Elektrode $23_3$ und zwei negativen Elektroden, nämlich den Elektroden $22_2$ und $22_3$. Beide Elektrodensätze sind mit einem gemeinsamen Generator 40 verbunden. Bei Bedarf kann auch jedem der Elektrodensätze ein separater Generator 40 zugeordnet sein.

Die zueinandergerichteten Stirnseiten der Elektroden $22_1$, $23_1$ und $23_2$ bzw. $22_2$, $22_3$ und $23_3$ sind hier verbunden durch einen weiteren isolierenden Elektrodenhalter 49. Elektrisch gesehen unteilt dieser die Elektroden-Vorrichtung 20 in zwei völlig voneinander getrennte Hälften. Bei der Elektroden-Vorrichtung 20 der Fig. 3 stehen demnach die Koaxial-Räume sowohl in Reihenals auch in Parallelschaltung zueinander, während in elektrischer Hinsicht sowohl zwei parallel geschaltete als auch hintereinander geschaltete Elektroden vorliegen. Durch die entgegengesetzte Polung der hintereinanderliegenden Elektroden-Gruppen stellen sich in der Elektroden-Vorrichtung 20 der Fig. 3 zwei unterschiedliche Feldstärkenrichtungen ein, die nacheinander auf die zu behandelnde Flüssigkeit (mit Bakterien) einwirken.

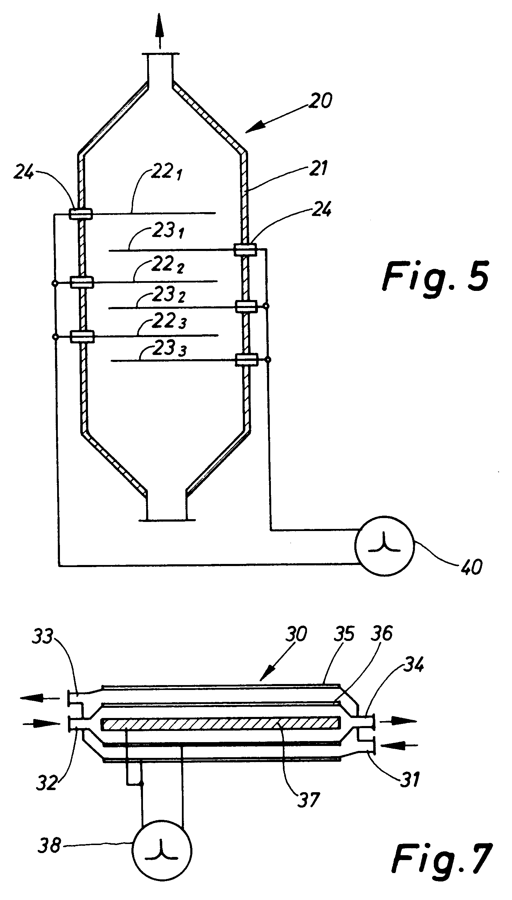

Bei der in Fig. 4 gezeigten Ausführungsform der Erfindung weisen die Elektroden $22_1$ - $22_3$ und $23_1$ - $23_3$ Durchlässe 25 auf und sind hintereinander quer zur Strömungsrichtung im Gehäuse 21 eingebaut. In elektrischer Hinsicht sind die Elektroden wiederum parallelgeschaltet und stehen mit einem Generator 40 in Verbindung.

Bei der in Fig. 5 ebenfalls im Längsschnitt gezeigten Vorrichtung sind die Elektroden $22_1$ - $22_3$ und $23_1$-$23_3$ ebenfalls elektrisch parallelgeschaltet, strömungsmäßig gesehen liegen sie jedoch in Form eines Labyrinthes hintereinander im Gehäuse 21, durch das sie über Isolierstücke 24 gelagert bzw. hindurchgeführt sind. Bei dieser Schaltung ergibt sich ebenso wie in Fig. 4 gezeigt eine längere Durchströmung eines Elektrodenzwischenraums.

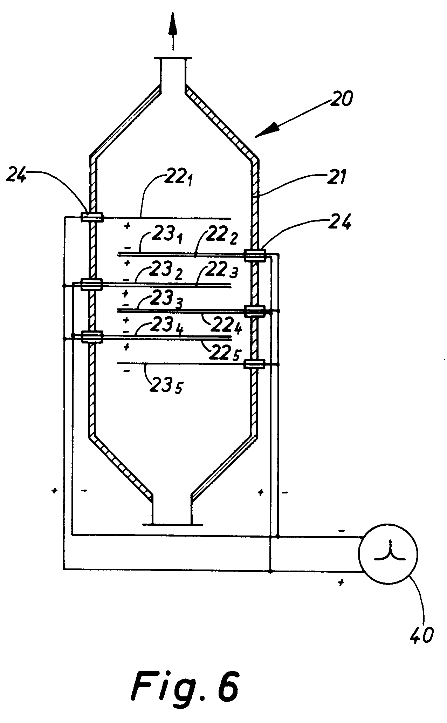

Die Fig. 6 zeigt die Elektroden-Vorrichtung 20 gemäß der Fig. 5 mit wiederum gruppenweise einander zugeordneten Elektroden. Abgesehen von den oberen und unteren Elektroden $22_1$ bzw. $23_5$ sind die übrigen Elektroden als Doppelelektroden $23_1$, $22_2$; $23_2$, $22_3$; $23_3$, $24_4$; $23_4$, $22_5$ ausgebildet. Sie sind durch in der Fig. 6 nicht dargestellte Materialien aus nicht gleitendem Werkstoff gegeneinander isoliert.

Sämtliche Elektroden werden auch hier wiederum von einem gemeinsamen Generator 40 aus gespeist. Die Schaltung derselben erfolgt dabei derart, daß entgegengesetzt polarisierte Elektroden den Strömungsweg der Flüssigkeit durch die Elektroden-Vorrichtung 20 begrenzen. Dadurch wirkt in jedem Labyrinthstrang die Feldstärke aus einer anderen Richtung auf die mit Bakterien angereicherte Flüssigkeit ein.

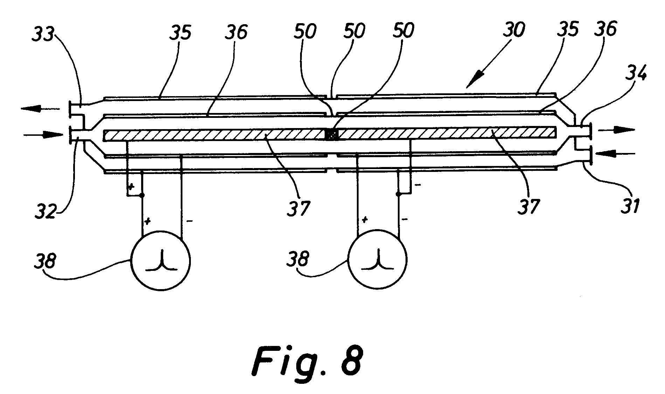

In Fig. 7 ist eine bevorzugte Ausführungsform der Elektrodenstrecke 30 gezeigt. Die Elektrodenstrecke 30 ist hier mit einer Doppelfunktion ausgestattet, ist nämlich zugleich Wärmetauscher und Entkeimungsvorrichtung.

An entgegengesetzten Enden der Elektrodenstrecke 30 liegen jeweils zwei Einlässe 31/33 sowie an ebenfalls entgegengesetzten Enden zwei Auslässe 32/34. Die Elektrodenstrecke 30 verfügt über einen äußeren Mantel 35, einen Zwischenmantel 36 und eine zentrale Elektrode 37. Der Zwischenmantel 36 liegt zwischen einem inneren strömungsmäßig abgeschlossenen Raum und dem äußeren, ebenfalls strömungsmäßig abgeschlossenen Raum.Die Durchströmung

erfolgt im Gegenstrom über den Zwischenmantel 36. Die Innenelektrode 37 und der Außenmantel 35 sind elektrisch miteinander verbunden, der Zwischenmantel 36 bildet zu beiden Elektroden die zweite Elektrode. Dieses Elektrodenpaar ist mit einem Sterilisier-Impulsgenerator 38 verbunden, der Pulse hoher Feldstärke auf die Platten führt.

Die Fig. 8 zeigt eine Weiterbildung der in Fig. 7 gezeigten Elektrodenstrecke 30, bei der wiederum zwei Elektrodensätze hintereinanderliegend vorgesehen sind. Abgegrenzt sind die einzelnen Eletrodensätze durch in der Fig. 8 lediglich schematisch dargestellt Isolierungen 50. Dadurch liegen bei der Elektrodenstrecke 30 jeweilse zwei Elektroden 35, 36 bzw. 37 in Reihe hintereinander. Die hintereinanderliegenden Elektroden sind dabei jeweils unterschiedlich gepolt. Im Ausführungsbeispiel der Fig. 8 sind in der linken Hälfte der Elektrodenstrecke 30 die Elektroden 35 und 37 positiv gepolt, während die Elektroden 36 negativ gepolt ist. Hingegen sind in der rechten Hälfte der Elektrodenstrecke 30 die Elektroden 35 und 37 negativ gepolt, während die dazwischenliegende Elektrode 36 hier positiv gepolt ist.

Im vorliegenden Ausführungsbeispiel ist jedem Elektrodensatz ein separater Sterilisier-Impulsgenerator 38 zugeordnet. Es ist alternativ allerdings auch möglich, beide Elektrodensätze durch einen gemeinsamen Sterilisier-Impulsgenerator 38 zu versorgen.

Im folgenden wird die elektrische Schaltung des zur Durchführung des erfindungsgemäßen Verfahrens notwendigen Impulsgenerators näher beschrieben. Dieser weist ein Netzteil 42 auf, das in üblicher Weise aus einem Netzschalter, einem Transformator, einem Gleichrichter und einem nachgeschalteten Glättungskondensator besteht. Das Netzteil 42 steht mit einem Pol eines Umschalters 43 in Verbindung, der über einen Steuergenerator 41 umgeschaltet werden kann. Der andere Pol des Umschalters 43 steht mit einer Elektrode 23 in Verbindung. Der Mittelpol des Umschalters 43 steht mit dem einen Pol eines Ladekondensators 44 in Verbindung, dessen anderer Pol über einen zweiten Umschalter wahlweise mit dem Netzgerät 42 und der zweiten Kondensatorplatte 22 in Verbindung steht. Bei Betätigung des Umschalters 43 wird der Kondensator 44 in einer ersten Stellung mit dem Netzgerät 42 verbunden, während des Umschaltens vom Netzteil 42 getrennt und bei erfolgtem Umschalten mit den Elektroden 22/23 verbunden. Zwischen den beiden Phasen besteht somit eine Pause, so daß die Elektroden 22/23 nicht mit dem Netzteil 42 verbunden werden.

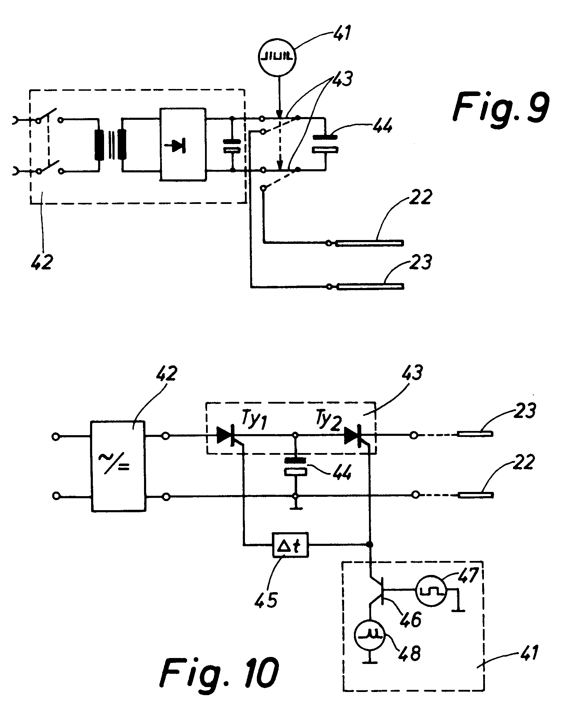

Bei der in Fig.10 gezeigten Ausführungsform der Erfindung besteht der Schalter 43 aus einem (einpoligen) Umschalter, der aus zwei Thyristoren gebildet wird. Der erste Thyristor $Ty_1$ ist zwischen das Netzgerät 42 und den Speicherkondensator 44 geschaltet, der zweite Thyristor (in gleicher Richtung gepolt) zwischen den Speicherkondensator 44 und die eine Elektrode 23. Die andere Elektrode 22 steht direkt mit dem anderen Ende des Speicherkondensators 44 und dem Netzteil 42 in Verbindung. Die Steuerleitung des ersten Thyristors $Ty_1$ ist über ein Verzögerungsglied 45 mit der Steuerleitung des zweiten Thyristors $Ty_2$ verbunden. Dieser Verbindungspunkt liegt auf dem einen Pol eines (Schalt-) Feldeffekttransistors 46, dessen anderer Pol auf dem Ausgang eines Pulsgenerators 48 liegt. Das GATE des Feldeffekttransistors 46 liegt auf dem Ausgang eines weiteren Generators 47. Diese Anordnung bildet den Steuerpulsgenerator 41. Gibt der Pulsgenerator 48 einen Puls an seinen Ausgang, und befindet sich zu diesem Zeitpunkt der Ausgang des weiteren Generators 47 auf hohem Pegel, so ist der Feldeffektransistor 46 leitend und der zweite Thyristor $Ty_2$ wird gezündet. Daraufhin entläd sich die im Kondensator 44 gespeicherte Ladung über die Platten 22/23 und den zwischen diesen Platten befindlichen Elektrolyten (Reaktorinhalt). Sobald sich der Kondensator 44 entladen hat, hört der Stromfluß durch den Thyristor $Ty_2$ auf, der Thyristor sperrt somit selbsttätig. Die Zeitkonstante des Verzögerungsgliedes 45 ist so gewählt, daß nun - nach Entladung des Kondensators 44 über die Elektroden 22/23 - der erste Thyristor $Ty_1$ zündet. Nach Zündung dieses ersten Thyristors $Ty_1$ wird der Kondensator 44 nun über das Netzteil 42 geladen. Sobald der Kondensator 44 geladen ist, hört der Stromfluß durch den ersten Thyristor $Ty_1$ auf, der Thyristor sperrt selbsttätig, und der Zyklus kann von neuem beginnen.

Die in den Fig. 9 und 10 gezeigte elektrische Schaltung zur Durchführung des erfindungsgemäßen Verfahrens kann gegebenenfalls noch derart modifiziert sein, daß diese über eine periodisch arbeitende, nicht gezeigte Umschalteinrichtung verfügt, mit der die Elektroden 22 und 23 abwechselnd umgepolt werden können zur Erzielung einer periodischen Umkehr der Richtung des elektrischen Feldes. Mit einem solchen Impulsgenerator lassen sich bei der Elektroden-Vorrichtung 20 der Fig.2 und 5 sowie der Elektrodenstrecke 30 der Fig. 7 abwechselnd unterschiedlich gerichtete elektrische Felder erzeugen, und zwar ohne daß es erforderlich wird, mehrere Elektrodensätze hintereinander anzuordnen, wie dies die Fig. 3, 6 und 8 z.B. zeigen. Dadurch wird auch eine Feldumkehr bei kleineren, kompakteren Anlagen in einfachster Weise ermöglicht.

**Patentansprüche**

1. Verfahren zur stoffwechsel- und/oder wachstumssteigernden Behandlung Von Mikroorganismen, wobei die Mikroorganismen elektrischen Feldern mit einer impulsförmig verlaufenden Feldstärke (E-Impulse) von bis zu 3,5 kV/cm ausgesetzt werden, **dadurch gekennzeichnet,** daß die Feldstärke der elektrischen Felder innerhalb von höchstens 1 ms auf den Maximalwert ansteigt und exponentiell abfällt.

2. Verfahren nach Anspruch 1, dadurch gekennzeichnet, daß die Feldstärke innerhalb von 10 $\mu$s auf den Maximalwert ansteigt und innerhalb von 10 bis 100 $\mu$s exponentiell abfällt.

3. Verfahren nach einem der vorhergehenden Ansprüche, dadurch gekennzeichnet, daß man die Feldstärke bis auf 0,1 - 1 kV/cm, vorzugsweise auf 0,2 - 0,5 kV/cm ansteigen läßt.

4. Verfahren nach einem der vorhergehenden Ansprüche, dadurch gekennzeichnet, daß die Feldstärkenrichtung in bezug auf die Strömungsrichtung der zu behandelnden Flüssigkeit (mit Bakterien) gewechselt wird.

5. Verfahren nach einem der vorhergehenden Ansprüche, dadurch gekennzeichnet, daß man die E-Impulse mit Repetitionsraten anwendet, die niedrig - gemessen an der Impulsdauer - sind, insbesondere zwischen 5 und 15 Hz liegen.

6. Verfahren nach einem der vorhergehenden Ansprüche, dadurch gekennzeichnet, daß man die E-Impulse in Pulsgruppen mit dazwischen liegenden Pausen anwendet, wobei die Pulsgruppen- bzw. Pausenintervalle kürzer als jeweils 30 Min., vorzugsweise kürzer als 15 Min. sind.

7. Verwendung des Verfahrens nach einem oder mehreren der Ansprüche 1 bis 6 zur mikrobiellen Erzeugung von gasförmigen Produkten, wie z.B. Biogas (Methan).

8. Verwendung des Verfahrens nach einem oder mehreren der Ansprüche 1 bis 6 zur mikrobiologischen Erzeugung von festen und/oder flüssigen Produkten, wie z.B. Enzymen.

9. Vorrichtung zur Durchführung des Verfahrens mach einem der vorhergehenden Ansprüche, mit mindestens einem Reaktor (10), der eine Zulaufleitung (12) und eine Ablaufleitung (13) aufweist, gekennzeichnet durch mindestens ein Paar von Elektroden (22, 23), die derart angeordnet sind, daß sich mindestens Teilmengen des sich im Reaktor (10) befindlichen Inhalts wenigstens zeitweise zwischen den Elektroden (22, 23) befinden, und durch eine impulserzeugende Generatorschaltung (40) mit mindestens einem Speicherkondensator (44) und einem Netzteil (42) zum Versorgen der Elektroden (22, 23) mit elektrischer Spannung, wobei der Speicherkondensator (44), das Netzteil (42) bzw. die Elektroden (22, 23) derart mit einem Umschalter (43) verschaltet sind, daß je nach Schalterstellung entweder der Speicherkondensator (44) geladen oder über die Elektroden (22, 23) entladen wird.

10. Vorrichtung nach Anspruch 9, dadurch gekennzeichnet, daß der Speicherkondensator (44) bzw. die Speicherkondensatoren (44) elektrisch steuerbar ausgebildet sind.

11. Vorrichtung nach Anspruch 9 oder 10, dadurch gekennzeichnet, daß der Umschalter (43) mit einem Steueroszillator (41) in gesteuerter Verbindung steht, dessen Ausgangspole vorzugsweise über Steuermittel (46) von einem Generator (47) periodisch steuerbar sind.

12. Vorrichtung nach einem oder mehreren der Ansprüche 9 bis 11, dadurch gekennzeichnet, daß der Umschalter (43) mindestens zwei An/Aus-Schaltelemente ($Ty_1$, $Ty_2$) aufweist, wobei das erste Schaltelement ($Ty_1$) zwischen Speicherkondensator (44) und Netzteil (42), das zweite Schaltelement ($Ty_2$) zwischen Speicherkondensator (44) und Elektroden (22, 23) angeordnet ist.

13. Vorrichtung nach Anspruch 9, dadurch gekennzeichnet, daß die Elektroden (22, 23) in einem Strommäßig separaten Gehäuse (21) angeordnet sind und daß der Zwischenraum zwischen den Elektroden (22, 23) über Leitungen (17, 18) in Strömungsverbindung mit dem Reaktorinnenraum in Verbindung steht, wobei vorzugsweise in einer der Leitungen (17) eine Umwälzpumpe (15) angeordnet ist.

14. Vorrichtung nach Anspruch 9 oder 13, dadurch gekennzeichnet, daß mehrere Elektroden ($22_1$ - $22_3$; $23_1$ - $23_3$) strömungsmäßig hintereinandergeschaltet sind.

15. Vorrichtung nach einem der Ansprüche 9 - 14, dadurch gekennzeichnet, daß die Elektroden ($22_1$ - $22_5$; $23_1$ - $23_5$) im Gehäuse (21) labyrinthartig angeordnet sind.

16. Vorrichtung nach einem der Ansprüche 9 - 15, dadurch gekennzeichnet, daß die Elektroden (22, 23) koaxial zueinander angeordnet sind.

17. Vorrichtung nach einem der Ansprüche 9 - 16, dadurch gekennzeichnet, daß die Elektroden ($22_1$ - $22_3$; $23_1$ - $23_3$) Durchlässe (25) aufweisen und derart im Gehäuse (21) angeordnet sind, daß sie im wesentlichen senkrecht zu ihren Oberflächen durchströmt werden.

18. Vorrichtung nach einem der Ansprüche 9 - 17, dadurch gekennzeichnet, daß die Zulauf- und die Ablaufleitung (12/13) über eine gemeinsame Elektrodenstrecke (30) und/oder eine Entkeimungsvorrichtung geführt sind.

19. Vorrichtung nach Anspruch 18, dadurch gekennzeichnet, daß die Elektrodenstrecke (30) mindestens ein Paar von (Kondensator-)Elektroden (35, 36, 37) aufweist, die derart angeordnet sind, daß die Elektrodenzwischenräume durchströmt werden können und daß die Elektroden (35 - 37) mit einer (weiteren) impulserzeugenden Generatorschaltung in Verbindung stehen, deren Puls-Ausgangsspannung steil ansteigt, aber exponentiell abfällt, und eine Spitzenamplitude von mehr als 3,5 kV/cm aufweist.

## Claims

1. Method of treating microorganisms to stimulate metabolism and/or growth, in which the microorganisms are exposed to electric fields with a pulse-type variation in field strength (E-pulses) of up to 3.5 kV/cm, characterised in that the field strength of the electric fields rises to the maximum value and decays exponentially within not more than 1 ms.

2. Method according to Claim 1, characterised in that the field strength rises to the maximum value within 10 μs and decays exponentially within 10 to 100 μs.

3. Method according to one of the preceding claims, characterised in that the field strength is caused to rise to 0.1 - 1 kV/cm, preferably to 0.2 - 0.5 kV/cm.

4. Method according to one of the preceding claims, characterised in that the field strength direction is varied in relation to the flow direction of the liquid (containing bacteria) to be treated.

5. Method according to one of the preceding claims, characterised in that the E-pulses are applied at repetition rates which are low, in particular between 5 and 15 Hz, when measured on the basis of the pulse duration.

6. Method according to one of the preceding claims, characterised in that the E-pulses are applied in groups of pulses with intervening breaks, the groups of pulses or the break periods each being shorter than 30 min, preferably shorter than 15 min.

7. Use of the method according to one or more of Claims 1 to 6 for the microbial production of gaseous products such as, for example, fermentation gas (methane).

8. Use of the method according to one or more of Claims 1 to 6 for the microbiological production of solid and/or liquid products such as, for example, enzymes.

9. Apparatus for carrying out the method according to one of the preceding claims, comprising at least one reactor (10) which has a feed line (12) and a drain line (13), characterised by at least one pair of electrodes (22, 23) which are arranged in such a way that at least portions of the contents contained in the reactor (10) are situated between the electrodes (22, 23) at least intermittently, and by a pulse-generating generator circuit (40) comprising at least one storage capacitor (44) and one power supply unit (42) for supplying the electrodes (22, 23) with electric voltage, the storage capacitor (44), the power supply unit (42) and the electrodes (22, 23) being connected to a changeover switch (43) in such a way that, depending on the position of the switch, the storage capacitor (44) is either charged or discharged via the electrodes (22, 23).

10. Apparatus according to Claim 9, characterised in that the storage capacitor (44) or the storage capacitors (44) is or are of electrically controllable design.

11. Apparatus according to Claim 9 or 10, characterised in that the changeover switch (43) is connected in a controlled manner to a controlling oscillator (41) whose output poles can be cyclically controlled, preferably via control means (46) of a generator (47).

12. Apparatus according to one or more of Claims 9 to 11, characterised in that the changeover switch (43) comprises at least two on/off switching devices ($Ty_1$, $Ty_2$), the first switching

device (Ty$_1$) being disposed between storage capacitor (44) and power supply unit (42) and the second switching device (Ty$_2$) being disposed between storage capacitor (44) and electrodes (22, 23).

13. Apparatus according to Claim 9, characterised in that the electrodes (22, 23) are disposed in a separate housing (21) in relation to the flow, and in that the gap between the electrodes (22, 23) is in flow connection with the reactor interior via lines (17, 18), a circulating pump (15) preferably being disposed in one of the lines (17).

14. Apparatus according to Claim 9 or 13, characterised in that a plurality of electrodes (22$_1$ - 22$_3$; 23$_1$ -23$_3$) are connected one behind the other in relation to the flow.

15. Apparatus according to one of Claims 9 - 14, characterised in that the electrodes (22$_1$ - 22$_5$; 23$_1$ - 23$_5$) are disposed in labyrinth fashion in the housing (21).

16. Apparatus according to one of Claims 9 - 15, characterised in that the electrodes (22, 23) are arranged coaxially one with respect to the other.

17. Apparatus according to one of Claims 9 - 16, characterised in that the electrodes (22$_1$ - 22$_3$; 23$_1$ - 23$_3$) have passages (25) and are arranged in the housing (21) in such a way that flow takes place through them essentially perpendicularly to their surfaces.

18. Apparatus according to one of Claims 9 - 17, characterised in that the feed line and drain line (12/13) are routed via a common electrode section (30) and/or a sterilising apparatus.

19. Apparatus according to Claim 18, characterised in that the electrode section (30) comprises at least one pair of (capacitor) electrodes (35, 36, 37) which are disposed in such a way that flow can take place through the electrode gaps, and in that the electrodes (35 - 37) are connected to a (further) pulse-generating generator circuit whose pulsed output voltage rises steeply but decays exponentially and has a peak amplitude higher than 3.5 kV/cm.

**Revendications**

1. Procédé pour le traitement de micro-organismes par augmentation du métabolisme et/ou de la croissance, dans lequel les micro-organismes sont exposés à des champs électriques d'une intensité de champ s'écoulant sous forme d'impulsions (impulsions E) allant jusqu'à 3,5 kV/cm, caractérisé en ce que l'intensité du champ électrique augmente jusqu'à la valeur maximum et diminue exponentiellement en au plus 1 ms.

2. Procédé suivant la revendication 1, caractérisé en ce que l'intensité de champ augmente en 10 $\mu$s jusqu'à la valeur maximum et diminue exponentiellement en 10 à 100 $\mu$s.

3. Procédcé suivant l'une quelconque des revendications précédentes, caractérisé en ce qu'on laisse l'intensité de champ augmenter jusqu'à 0,1 à 1 kV/cm, de préférence jusqu'à 0,1 à 0,5 kV/cm.

4. Procédé suivant l'une quelconque des revendications précédentes, caractérisé en ce que le sens d'intensités de champ est alterné par rapport au sens d'écoulement du liquide (avec bactéries) à traiter.

5. Procédé suivant l'une quelconque des revendications précédentes, caractérisé en ce qu'an applique les impulsions E avec des taux de répétition qui, mesurés à la durée d'impulsion, sont faibles, et en particulier sont situés entre 5 et 15 Hz.

6. Procédé suivant l'une quelconque des revendications précédentes, caractérisé en ce qu'on applique les impulsions E en groupes d'impulsions avec des poses situées entre ceux-ci, les groupes d'impulsions ou respectivement les intervalles de poses étant plus courts chaque fois que 30 minutes, et de préférence que 15 minutes.

7. Utilisation du procédé suivant une ou plusieurs des revendications 1 à 6 pour la production de produits gazeux par fermentation comme par exemple du gaz biologique (méthane).

8. Utilisation du procédé suivant une ou plusieurs des revendications 1 à 6 pour la production microbiologique de produits solides et/ou liquides comme par exemple des enzymes.

9. Dispositif pour la mise en oeuvre du procédé suivant l'une quelconque des revendications précédentes, comportant au moins un réacteur (10) qui présente une conduite d'arrivée (12) et une conduite d'évacuation (13), caractérisé par au moins une paire d'électrodes (22, 23), qui sont agencées de façon qu'au moins des quantités partielles du contenu se trouvant dans le réacteur (10) se trouvent au moins temporairement entre les électrodes (22, 23), et par un circuit de générateur (40) produisant des impulsions et comportant au moins un condensateur de stockage (44) et une partie de réseau (42) pour l'alimentation des électrodes (22, 23) en tension électrique, le condensateur de stockage (44), la partie de réseau (42) et respectivement les électrodes (22, 23) étant câblés avec un commutateur (43) de façon que, selon une position de commutateur, le condensateur de stockage (44) soit soit chargé soit déchargé entre les électrodes (22, 23).

10. Dispositif suivant la revendication 9, caractérisé en ce que le condensateur de stockage (44) ou respectivement les condensateurs de stockage (44) sont réalisés de façon à pouvoir être commandés électriquement.

11. Dispositif suivant l'une ou l'autre des revendications 9 et 10, caractérisé en ce que le commutateur (43) est en liaison commandée avec un oscillateur de commande (41) dont les poses de sortie peuvent être commandées périodiquement de préférence par des moyens de commande (46) à partir d'un générateur (47).

12. Dispositif suivant une ou plusieurs des revendications 9 à 11, caractérisé en ce que le commutateur (43) présente au moins deux éléments de commutation fermés/ouverts ($Ty_1$, $Ty_2$), le premier élément de commutation ($Ty_1$) étant agencé entre condensateur de stockage (44) et partie de réseau (42), le second élément de commutation ($Ty_2$) entre condensateur de stockage (44) et électrodes (22, 23).

13. Dispositif suivant la revendication 9, caractérisé en ce que les électrodes (22, 23) sont agencées dans un boîtier (21) séparé suivant le courant et en ce que l'espace intercalaire entre les électrodes (22, 23) est en liaison par des conduites (17, 18), dans un raccordement d'écoulement, avec l'espace interne de réacteur, de préférence une pompe de circulation (15) étant agencée dans une des conduites (17).

14. Dispositif suivant l'une quelconque des revendications 9 à 13, caractérisé en ce que plusieurs électrodes ($22_1$ à $22_3$, $23_1$ à $23_3$) sont connectées l'une derrière l'autre suivant l'écoulement.

15. Dispositif suivant l'une quelconque des revendications 9 à 14, caractérisé en ce que les électrodes ($22_1$ à $22_5$ ; $23_1$ à $23_5$) sont agencées en un genre de labyrinthe dans le boîtier (21).

16. Dispositif suivant l'une quelconque des revendications 9 à 15, caractérisé en ce que les électrodes (22, 23) sont agencées coaxialement l'une par rapport à l'autre.

17. Dispositif suivant l'une quelconque des revendications 9 à 16, caractérisé en ce que les électrodes ($22_1$ à $22_3$ ; $23_1$ à $23_3$) présentent des passages (25) et sont agencées dans le boîtier (21) de façon qu'elles soient traversées sensiblement perpendiculairement à leurs surfaces.

18. Dispositif suivant l'une quelconque des revendications 9 à 17, caractérisé en ce que les conduites d'arrivée et d'évacuation (12, 13) sont conduites sur une section d'électrodes commune (30) et/ou sur un dispositif de stérilisation.

19. Dispositif suivant la revendication 18, caractérisé en ce que la section d'électrodes (30) présente au moins une paire d'électrodes (de condensateur) (35, 36, 37) qui sont agencées de façon que les espaces intercalaires entre électrodes puissent être parcourus et en ce que les électrodes (35 à 37) soient en liaison avec un (autre) circuit de générateur de production d'impulsions dont la tension de sortie d'impulsions augmente de façon rapide mais tombe exponentiellement et présente une amplitude de crête de plus de 3,5 kV/cm.

**Fig. 1**

Fig. 2

Fig. 4

Fig. 3

*Fig. 5*

*Fig. 7*

14

Fig. 6

*Fig. 8*

Fig.9

Fig. 10

17